# EUROPÄISCHE PATENTANMELDUNG

(11) **EP 3 816 607 A1**
(43) Veröffentlichungstag der Anmeldung: **05.05.2021**
(21) Anmeldenummer: 20201924.6
(22) Anmeldetag: 15.10.2020
(51) Int. Cl.: G01N 11/14, G01N 33/38

(54) **RHEOLOGIELANZE**

(30) Priorität: 31.10.2019 DE 102019216892
(71) Anmelder: KNIELE GmbH, 88422 Bad Buchenau (DE)
(72) Erfinder: GÜNTHNER, Christoph, 88348 Bad Saulgau (DE)
(74) Vertreter: Prüfer & Partner mbB Patentanwälte · Rechtsanwälte

(57) **Zusammenfassung**

Die vorliegende Erfindung bezieht sich auf eine Rheologiemessvorrichtung (1), welche als Rheologielanze ausgeführt ist. Die Rheologiemessvorrichtung (1) beinhaltet ein Rheologiemesswerkzeug (2), bestehend aus einem Messkörper (2a) sowie einer Begrenzungsvorrichtung (2b), einem Motor (3), welcher mit dem Messkörper (2a) verbunden ist und dazu angepasst ist, eine Rotationsbewegung auf diesen zu übertragen, einem Drehmomentsensor (4), welcher dazu angepasst ist, das Drehmoment zu messen, mit welchem der Messkörper (2a) durch den Motor (3) beaufschlagt wird, einem Drehzahlsensor (5), welcher dazu angepasst ist, die Drehzahl des Messkörpers (2a) zu messen, und einem Abstandssensor (6), welcher dazu angepasst ist, den Abstand zu einem zu messenden Mischgut zu messen.

Die Position des Rheologiemesswerkzeugs (2) relativ zum Mischgut ist durch einen Linearantrieb (7) einstellbar, und somit ist die Eintauchtiefe des Messkörpers (2a) in das Mischgut veränderbar.

Eine solche Rheologiemessvorrichtung ermöglicht es, bestehende Mischer und Mischersysteme um die Möglichgeit einer rheologischen Messung zu erweitern, welche bei jeder Charge durchgeführt werden kann und somit eine gleichbleibende Qualität ermöglicht wird.

## Beschreibung

Die vorliegende Erfindung bezieht sich auf eine Rheologiemessvorrichtung in Form einer Rheologielanze, welche rheologische Messungen eines Mischguts während eines Mischvorgangs ermöglichen soll.

Besonders in der Baustoff- und Betonindustrie ist die Vorhersage von Eigenschaften von Mischgut äußerst wichtig, da die Bandbreite der Anforderungen an verschiedene Betone in vergangener Zeit stetig gestiegen ist. Insbesondere die rheologischen Eigenschaften sind hierbei sehr wichtig, da Betone für unterschiedliche Anforderungen benötigt werden und somit verschiedene rheologische Eigenschaften nötig sein können. Rheologische Messungen können nicht nur nach einem Mischvorgang, sondern auch während einem Mischvorgang, beispielsweise zwischen zwei Mischungszyklen notwendig sein - beispielsweise, wenn sich das zu vermischende Gut nach dem Mischvorgang schnell wieder entmischt, oder wenn überlegt werden muss, ob zur Mischung noch weitere Substanzen hinzugegeben werden müssen, um eine bestimmte rheologische Eigenschaft zu erreichen.

Aus dem Stand der Technik ist das Dokument DE202010005289U1 bekannt, welches eine Rheologiemessvorrichtung mit zwei relativ zueinander verdrehbaren Platten offenbart. Ein zu vermessendes Mischgut kann in einen Messspalt zwischen den Platten eingebracht werden, wobei ein Anpressdruck von der einen Platte über das Mischgut auf die andere Platte übertragbar ist. Beide Platten sind drehbar. Mit Hilfe einer Mess- und Auswerteeinrichtung können charakteristische Fließparameter des zu vermessenden Mischguts sowie eine charakteristische Fließkurve des Mischguts bestimmt werden.

Diese Rheologiemessvorrichtung ist jedoch nicht in eine Mischvorrichtung integrierbar - vielmehr muss aus einer Mischvorrichtung eine Probe entnommen werden, von welcher dann die rheologischen Eigenschaften bestimmt werden können.
Aus dem Stand der Technik sind ferner die Dokumente DE 10 2015 225 274 B3 sowie DE 10 2015 225 277 B4 bekannt, welche eine Vorrichtung, in welcher ein Mischvorgang und ein Rheologiemessvorgang realisiert werden können, und ein kombiniertes Misch- und Rheologiemesswerkzeug zeigen.
Hier sind an einen Apparat sowohl ein Mischwerkzeug als auch ein Werkzeug für die Rheologiemessung anbringbar, und es können in einer Apparatur sowohl ein Mischvorgang durchgeführt werden als auch eine Rheologiemessung durchgeführt werden. Die Werkzeuge zum Mischen sowie zur Rheologiemessung eignen sich jeweils nur für einen Vorgang und müssen ausgetauscht werden, wenn der jeweils andere Vorgang durchgeführt werden soll.

Der Nachteil der Systeme im Stand der Technik ist allerdings, dass bestehende Mischsysteme nur schwer mit entsprechenden Rheologiemesswerkzeugen nachgerüstet werden können.

Es ist daher die Aufgabe der vorliegenden Erfindung, rheologische Messungen eines Mischguts auch während eines Mischvorgangs durchführen zu können und somit bestehende Mischsysteme einfach und kostengünstig mit einem Rheologiemesswerkzeug auszurüsten.

Diese Aufgabe wird gelöst durch eine Rheologiemessvorrichtung gemäß Anspruch 1, ein Rheologiemessverfahren gemäß Anspruch 10 oder 11 sowie eine Verwendung einer Rheologiemessvorrichtung gemäß Anspruch 13.
Weitere vorteilhafte Ausgestaltungen sind Gegenstand der abhängigen Ansprüche.

Die vorliegende Erfindung umfasst eine Rheologiemessvorrichtung mit einem Rheologiemesswerkzeug, das typischerweise aus einem Messkörper sowie einer Begrenzungsvorrichtung besteht. Ferner ist ein Motor enthalten, welcher mit dem Messkörper verbunden ist und auf diesen eine Rotationsbewegung übertragen kann. Ferner ist ein Drehmomentsensor vorgesehen, welcher dazu angepasst ist, ein Drehmoment zu messen, mit welchem der Messkörper durch den Motor beaufschlagt wird. Ferner sind ein Drehzahlsensor, welcher dazu angepasst ist, die Drehzahl des Messkörpers zu messen, sowie ein Abstandssensor, der dazu angepasst ist, den Abstand zu einem zu messenden Mischgut zu messen, vorgesehen.

Die Position des Rheologiemesswerkzeugs relativ zum Mischgut ist einstellbar, vorzugsweise durch einen Linearantrieb, und somit ist die Eintauchtiefe des Messkörpers in das Mischgut veränderbar.

Eine solche Rheologiemessvorrichtung ermöglicht es, bestehende Mischer und Mischersysteme um die Möglichgeit einer rheologischen Messung zu erweitern, welche bei jeder Charge durchgeführt werden kann und somit eine gleichbleibende Qualität ermöglicht wird. Je nach Bauart der Mischer kann der Mischprozess sogar parallel zur Rheologiemessung weiter ablaufen. Durch die automatisierte Einstellung der Eintauchtiefe des Messkörpers in das Mischgut ermöglich eine solche Rheologiemessvorrichtung noch genauere und noch besser reproduzierbare Ergebnisse. Auch besteht die Möglichkeit, wiederholte Messungen durchzuführen, beispielsweise nach mehreren Mischzyklen - dann können, durch ein automatisiertes und präzises Einfahren der Rheologiemessvorrichtung, besonders gut vergleichbare Meßwerte erhalten werden.
Ferner ermöglicht es eine solche Rheologiemessvorrichtung, dass verschiedene Füllstände eines Mischgefäßes automatisiert berücksichtigt werden können.

Vorzugsweise weist die Rheologiemessvorrichtung einen Rahmen auf. Dieser begrenzt die Rheologiemessvorrichtung, und ermöglicht ferner ein festes Anbringen der Rheologiemessvorrichtung an einen Mischbehälter.

Weiter vorzugsweise weist die Rheologiemessvorrichtung einen Träger auf, an welchem wichtige Komponenten der Rheologiemessvorrichtung angebracht sind (Rheologiemesswerkzeug, Motor, Drehmomentsensor, Abstandssensor sowie Linearantrieb, die fest mit dem Träger verbunden sind). Die Position des Trägers ist relativ zum Rahmen durch den Linearantrieb veränderbar. Dies erlaubt eine kompakte Bauweise, und ferner wird sichergestellt, dass der Messkörper und der Abstandssensor sich gleichzeitig bewegen. Auf diese Art und Weise ist es ferner möglich, dass die Rheologiemessvorrichtung in ein Mischgut eingetaucht wird und sich dann mit Hilfe des Abstandssensors automatisch konfiguriert, so dass eine optimale Eintauchtiefe des Rheologiemesswerkzeugs in das zu mischende Gut bestimmt werden kann und auch realisiert werden kann. Die Rheologiemessvorrichtung, welche in Form einer Lanze ausgebildet ist, ist äußerst dünn und kann daher leicht in bestehende Mischsysteme eingebracht werden, ohne dass hierzu das Mischwerkzeug ausgebaut werden müsste - es reicht aus, das Mischwerkzeug anzuhalten.

Vorzugsweise weist der Rahmen an einem Ende ein Element, welches das Aufstellen der Rheologiemessvorrichtung ermöglicht, weiter vorzugsweise ein ringförmiges Element auf, mit welchem der Rahmen auf den Boden eines Mischbehälters gerade und stabil aufgestellt werden kann. Dies ermöglicht genauere und besser reproduzierbare Ergebnisse.

Ferner vorzugsweise weist die Rheologiemessvorrichtung eine Steuervorrichtung auf, welche dazu angepasst ist, basierend auf Messungen des Abstandssensors die Bewegung des Linearantriebs zu steuern. Somit kann eine optimale Eintauchtiefe des Messkörpers bestimmt werden, und der Messkörper kann automatisiert durch Bewegung des Linearantriebs bis zur optimalen Eintauchtiefe in das zu vermessende Mischgut eingefahren werden. Auch dies ermöglicht genauere und besser reproduzierbare Ergebnisse.

Weiter vorzugsweise ist die Rheologiemessvorrichtung mit einem Messkörper in Form eines Zylinders, Kegels oder Kegelstumpfs oder einer Kombination aus mindestens zwei dieser Formen vorgesehen, wodurch ein für das zu mischende Gut passender Messkörper vorgesehen werden kann.

Die Begrenzungsvorrichtung ist vorzugsweise in Form eines Hohlzylinders ausgeführt.

Vorzugsweise ist der Abstandssensor als optischer Sensor (hier vorzugsweise als Lasersensor) und als akustischer Sensor (hier vorzugsweise als Ultraschallsensor) vorgesehen. Der optische Sensor hat den Vorteil einer hohen Präzision, während ein akustischer Sensor den Vorteil hat, dass selbst bei Verunreinigung durch spritzendes Mischgut Messergebnisse nicht oder nur gering verfälscht werden und ein geringerer Platzbedarf vorhanden ist.
Weiter vorzugsweise sind mehrere (beispielsweise 2 bis 4) miteinander verschaltete Ultraschallsensoren vorgesehen, um eine Oberflächenebene zu errechnen

Ferner weist die Rheologiemessvorrichtung vorzugsweise eine Reinigungsvorrichtung auf, welche dazu angepasst ist, das Rheologiemesswerkzeug nach Verwendung zu reinigen, indem Wasser oder ein anderes Mischgut aus dieser Reinigungsvorrichtung ausgegeben wird.

Eine solche Reinigungsvorrichtung hat vorzugsweise mehrere Düsen, vorzugsweise Flachstrahldüsen oder Rundstrahldüsen oder eine Kombination hiervon, welche dazu geeignet sind, eine Reinigungsaufgabe schnell und zuverlässig zu erfüllen.

Ein drehmomentgesteuertes Rheologiemessverfahren gemäß der vorliegenden Erfindung enthält die folgenden Schritte:
a) Einbringen der Rheologiemessvorrichtung in ein zu vermessendes Mischgut,
b) Bewegen des Rheologiemesswerkzeugs in das zu vermessende Mischgut, wobei die Eintauchtiefe des Messkörpers in das zu messende Mischgut durch den Abstandssensor gesteuert wird,
c) Beaufschlagen des Messkörpers mit einem Drehmoment, welches durch den Motor (3) erzeugt wird,
d) Erhöhen des Drehmoments, bis sich der Messkörper zu drehen beginnt,
e) Erfassen der Drehbewegung des Messkörpers durch den Drehzahlsensor,
f) weiteres Erhöhen des Drehmoments, bis ein vorbestimmtes Messdrehmoment erreicht ist, dabei Aufzeichnen der Drehzahl des Messkörpers und des Drehmoments,
g) Bestimmen der rheologischen Eigenschaften des zu vermessenden Mischguts.

Während der Rotation des Messkörpers wird das benötigte Drehmoment gemessen. Daraus und aus der Drehgeschwindigkeit (sowie aus der exakten Geometrie des verwendeten Drehkörpers) wird die dynamische Viskosität der Flüssigkeit bestimmt. So kann beispielsweise die dynamische Viskosität eines Mischguts bestimmt werden (Verhältnis von Schubspannung und Geschwindigkeitsgradient). Auch ist die Fließgrenze eines Bingham-Fluids bestimmbar.

Ein drehzahlgesteuertes Rheologiemessverfahren gemäß der vorliegenden Erfindung enthält die folgenden Schritte:
a) Einbringen der Rheologiemessvorrichtung (1) in ein zu vermessendes Mischgut,
b) Bewegen des Rheologiemesswerkzeugs (2) in das zu vermessende Mischgut, wobei die Eintauchtiefe des Messkörpers (2a) in das zu vermessende Mischgut durch den Abstandssensor (6) gesteuert wird,
c) Rotieren des Messkörpers (2a) durch den Motor (3) mit einer Drehzahl,
d) Erfassen des Drehmoments des Messkörpers (2a) durch den Drehmomentsensor (4),
e) weiteres Erhöhen der Drehzahl, bis eine vorbestimmte Messdrehzahl erreicht ist, dabei Aufzeichnen der Drehzahl und des Drehmoments des Messkörpers (2a),
f) Bestimmender rheologischen Eigenschaften des zu vermessenden Mischguts.

Solche Verfahren ermöglichen eine Messung von rheologischen Eigenschaften in einem bestehenden Mischer oder Mischersystem, ferner ermöglicht es genauere und besser reproduzierbare Ergebnisse.

Vorzugsweise wird vor Schritt a) eine Leerlaufmessung durchgeführt, um verschleißbedinge Reibungsverluste bei den eigentlichen Messergebnissen berücksichtigen zu können.

Vorzugsweise enthalten die Rheologiemessverfahren weiterhin die folgenden Schritte:
h) Ausfahren der Rheologiemessvorrichtung (1) aus dem zu vermessenden Mischgut,
i) Reinigen der Rheologiemessvorrichtung (1) durch die Reinigungsvorrichtung (11).

So kann ein wirtschaftlicher Betrieb mit einem schnelleren Wiedereinsatz der Rheologiemessvorrichtung nach einer Messung garantiert werden.

In der Praxis kann es sich als Vorteil erweisen, dass bei der Aufnahme mehrerer Meßpunkte die Rheologielanze aus dem Mischer gefahren wird, um den Beton nochmals zwischenzumischen. Danach kann die Rheologiemessung fortgesetzt werden.

Die Rheologiemessvorrichtung kann für eine Rheologiemessung eines Mischguts in einem Mischer, vorzugsweise in einem Betonmischer verwendet werden. Für eine solche Anwendung ist diese Rheologiemessvorrichtung sehr gut geeignet und kann ihre Vorteile besonders gut entfalten.

Im Folgenden wird ein bevorzugtes Ausführungsbeispiel unter Bezugnahme auf die beigefügten Zeichnungen näher erläutert.
- Fig. 1: ist eine Draufsicht auf eine Rheologiemessvorrichtung gemäß der vorliegenden Erfindung.
- Fig. 2: ist eine Ansicht von unten auf eine Rheologiemessvorrichtung gemäß der vorliegenden Erfindung.
- Fig. 3: ist eine isometrische Ansicht einer Rheologiemessvorrichtung gemäß der vorliegenden Erfindung.

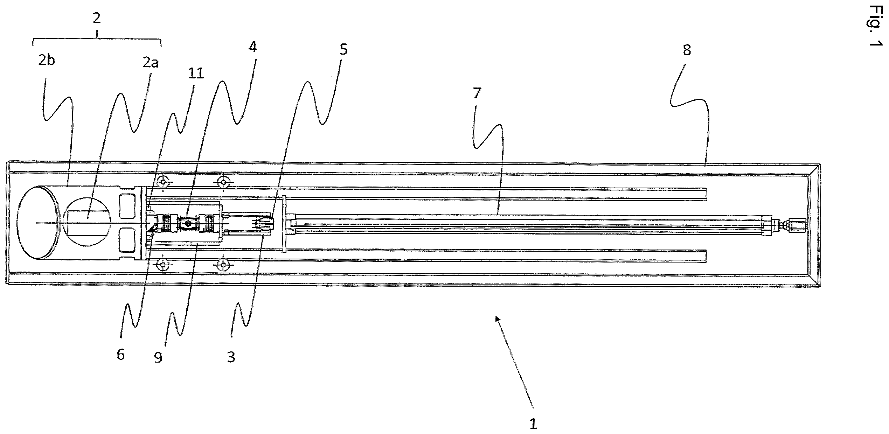

Fig. 1 zeigt eine Rheologiemessvorrichtung 1, welche eine lanzenförmige Form aufweist. Diese kann somit leicht in ein Mischgut eingeschoben werden. Die Rheologiemessvorrichtung 1 beinhaltet ein Rheologiemesswerkzeug 2, welches aus einem Messkörper 2a sowie einer Begrenzungsvorrichtung 2b besteht. Die Begrenzungsvorrichtung 2b ist hier ein hohler Zylinder, welcher hinsichtlich des Messkörpers 2 konzentrisch angeordnet ist. Der Messkörper 2 ist mit einem Motor 3 verbunden, welcher dazu angepasst ist, eine Rotationsbewegung auf den Messkörper 2a zu übertragen. Ferner vorgesehen sind ein Drehmomentsensor 4, welcher zwischen dem Motor 3 und dem Messkörper 2a angebracht ist und dazu angepasst ist, das Drehmoment zu messen, mit welchem der Messkörper 2a durch den Motor 3 beaufschlagt wird, und ein Drehzahlsensor 5, welcher dazu angepasst ist, die Drehzahl des Messkörpers 2a zu messen. Ferner ist in der Nähe des Messkörpers 2a ein Abstandssensor 6 angeordnet, welcher dazu angepasst ist, den Abstand zu einem zu messenden Mischgut zu messen. Das Rheologiemesswerkzeug 2, der Motor 3, der Drehmomentsensor 4 sowie der Abstandssensor 6 sind auf einem Träger 9 angeordnet. Dieser Träger 9 ist innerhalb eines Rahmens 8 beweglich angeordnet. Zwischen dem Träger 9 und einem Teil des Rahmens 8 ist ein Linearantrieb 7 angeordnet, welcher dazu angepasst ist, die Position des Trägers 9 relativ zum Rahmen 8 zu verändern. So ist es möglich, dass die gesamte Rheologiemessvorrichtung 1 in ein Mischgut bzw. zu vermessendes Mischgut eingestellt werden kann, beispielsweise mit der unteren Seite des Rahmens 8. Durch den Abstandssensor 6 kann der Abstand zur Flüssigkeitsoberfläche, d.h. dem zu messenden Mischgut, gemessen werden, und entsprechend kann die Position des Trägers 9 durch den Linearantrieb 7 verändert werden, so dass der Messkörper 2a in das zu messende Mischgut eintaucht. Ferner vorgesehen ist eine Reinigungsvorrichtung 11, welche hier ebenfalls auf dem Träger 9 angeordnet ist. Diese kann beispielsweise aus Düsen 12 (hier nicht dargestellt) bestehen, durch welche ein Mischgut, beispielsweise Wasser oder ein Luft-Wassergemisch eingespritzt werden kann, um eben den Messkörper 2a zu reinigen.
Das Rheologiemesssystem 1 umfasst ferner eine Steuervorrichtung 10 (hier nicht gezeigt), welche dazu angepasst ist, basierend auf Messungen des Abstandssensors 6 die Bewegung des Linearantriebs 7 zu steuern.

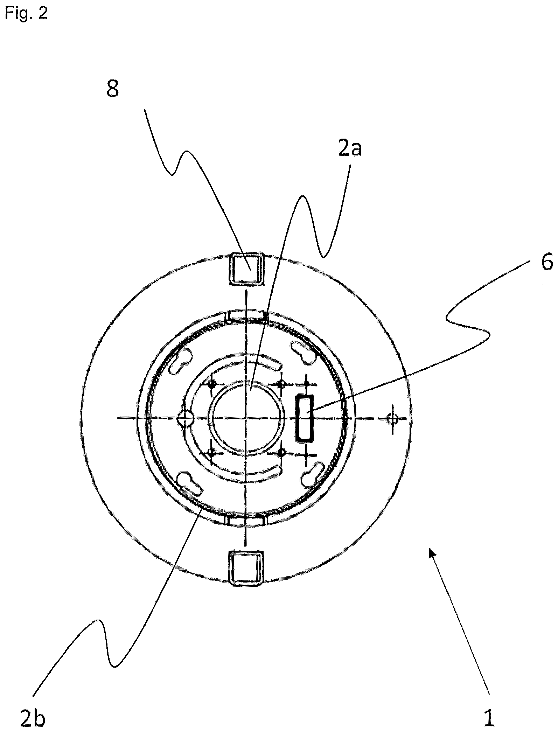

Fig. 2 ist eine Ansicht der Rheologiemessvorrichtung 1 von unten. Ersichtlich sind hier der Messkörper 2a sowie die Begrenzungsvorrichtung 2b. Ferner ist der Abstandssensor 6 ersichtlich, welcher somit direkt auf das zu mischende Gut blickt. Ebenfalls sind Teile des Rahmens 8 zu sehen.

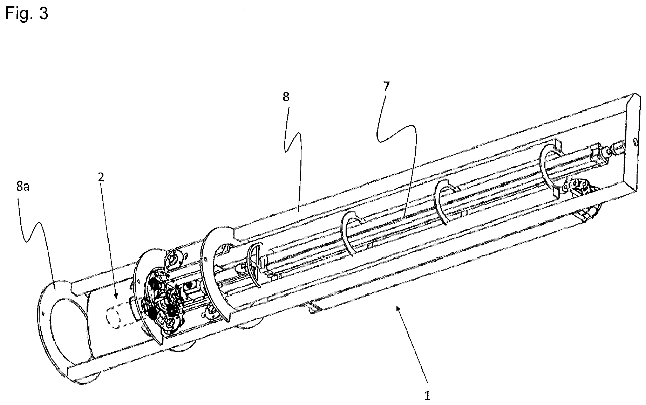

Fig. 3 zeigt eine isometrische Gesamtansicht des Systems, allerdings ohne Details des Rheologiemesswerkzeugs 2. Allerdings werden hier weitere Details des Rahmens 8 gezeigt - beispielsweise zeigt dieser an seinem unteren Ende noch ein ringförmiges Element 8a, mit welchem die gesamte Rheologiemessvorrichtung 1 auf den Boden eines Mischgefäßes aufgestellt werden kann. Somit steht die Rheologiemessvorrichtung immer auf dem Boden des Mischgefäßes auf, und somit ist ein sicherer Stand gewährleistet, so dass eine Verfälschung von Messergebnissen reduziert werden kann. Ferner ist gezeigt, dass der Linearantrieb 7 mit dem Rahmen 8 verbunden ist.

Die vorliegende Erfindung ist nicht auf die oben genannte Ausführungsform beschränkt:
Der Messkörper 2a kann jede beliebige Form aufweisen, sollte allerdings bevorzugt rotationssymmetrisch sein.

Der Abstandssensor 6 ist ebenfalls nicht auf einen optischen oder akustischen Sensor beschränkt. Hier könnte alternativ ein magnetischer Verschiebungssensor vorgesehen sein, welcher beispielsweise am Rahmen 8 oder am Träger 9 vorgesehen sein kann.

Ferner ist es auch möglich dass, der Motor 3 nicht am Träger 9 angeordnet ist. Der Motor 3 und der Messkörper 2a könnten alternativ auch durch eine Teleskopstange verbunden sein, und der Motor könnte somit alternativ fest am Rahmen 8 angeordnet sein.

Die vorliegende Erfindung bezieht sich auf eine Rheologiemessvorrichtung 1, welche als Rheologielanze ausgeführt ist. Die Rheologiemessvorrichtung 1 beinhaltet ein Rheologiemesswerkzeug 2, bestehend aus einem Messkörper 2a sowie einer Begrenzungsvorrichtung 2b, einem Motor 3, welcher mit dem Messkörper 2a verbunden ist und dazu angepasst ist, eine Rotationsbewegung auf diesen zu übertragen, einem Drehmomentsensor 4, welcher dazu angepasst ist, das Drehmoment zu messen, mit welchem der Messkörper 2a durch den Motor 3 beaufschlagt wird, einem Drehzahlsensor 5, welcher dazu angepasst ist, die Drehzahl des Messkörpers 2a zu messen, und einem Abstandssensor 6, welcher dazu angepasst ist, den Abstand zu einem zu messenden Mischgut zu messen.
Die Position des Rheologiemesswerkzeugs 2 relativ zum Mischgut ist durch einen Linearantrieb 7 einstellbar, und somit ist die Eintauchtiefe des Messkörpers 2a in das Mischgut veränderbar.
Eine solche Rheologiemessvorrichtung ermöglicht es, bestehende Mischer und Mischersysteme um die Möglichgeit einer rheologischen Messung zu erweitern, welche bei jeder Charge durchgeführt werden kann und somit eine gleichbleibende Qualität ermöglicht wird.

## Patentansprüche

1. Rheologiemessvorrichtung (1), welche aufweist:
ein Rheologiemesswerkzeug (2), bestehend aus einem Messkörper (2a) sowie einer Begrenzungsvorrichtung (2b),
einen Motor (3), welcher mit dem Messkörper (2a) verbunden ist und dazu angepasst ist, eine Rotationsbewegung auf diesen zu übertragen,
einen Drehmomentsensor (4), welcher dazu angepasst ist, das Drehmoment zu messen, mit welchem der Messkörper (2a) durch den Motor (3) beaufschlagt wird,
einen Drehzahlsensor (5), welcher dazu angepasst ist, die Drehzahl des Messkörpers (2a) zu messen,
und einen Abstandssensor (6), welcher dazu angepasst ist, den Abstand zu einem zu messenden Mischgut zu messen,
wobei die Position des Rheologiemesswerkzeugs (2) relativ zum Mischgut einstellbar ist und somit die Eintauchtiefe des Messkörpers (2a) in das Mischgut veränderbar ist.

2. Rheologiemessvorrichtung (1) gemäß Anspruch 1, wobei die Position des Rheologiemesswerkzeugs (2) relativ zum Mischgut durch einen Linearantrieb (7) einstellbar ist.

3. Rheologiemessvorrichtung (1) gemäß Anspruch 1 oder 2, ferner aufweisend:
einen Träger (9), wobei das Rheologiemesswerkzeug (2), der Motor (3), der Drehmomentsensor (4), der Abstandssensor (6) sowie der Linearantrieb (7) fest mit dem Träger (9) verbunden sind,
und die Position des Trägers (9) relativ zum Mischgut veränderbar ist.

4. Rheologiemessvorrichtung (1) gemäß einem der vorhergehenden Ansprüche, wobei ferner ein Rahmen (8) vorgesehen ist, der vorzugsweise an einem Ende ein Element (8a), welches das Aufstellen der Rheologiemessvorrichtung (1) ermöglicht, weiter vorzugsweise ein ringförmiges Element aufweist.

5. Rheologiemessvorrichtung gemäß einem der vorhergehenden Ansprüche, ferner eine Steuervorrichtung (10) aufweisend, welche dazu angepasst ist, basierend auf Messungen des Abstandssensors (6) die Bewegung des Linearantriebs (7) zu steuern.

6. Rheologiemessvorrichtung gemäß einem der vorhergehenden Ansprüche, wobei der Messkörper (2a) in Form eines Zylinders, Kegels oder Kegelstumpfs oder als Kombination mindestens zweier dieser Formen ausgebildet ist, und/oder die Begrenzungsvorrichtung (2b) in Form eines Hohlzylinders ausgeführt ist.

7. Rheologiemessvorrichtung gemäß einem der vorhergehenden Ansprüche, wobei der Abstandssensor (6) ein akustischer Sensor, vorzugsweise ein Ultraschallsensor, oder ein optischer Sensor, vorzugsweise ein Lasersensor, ist.

8. Rheologiemessvorrichtung gemäß einem der vorhergehenden Ansprüche, ferner eine Reinigungsvorrichtung (11) aufweisend, welche dazu angepasst ist, das Rheologiemesswerkzeug (2) zu reinigen.

9. Rheologiemessvorrichtung (1) gemäß Anspruch 8, wobei die Reinigungsvorrichtung (11) eine oder mehrere Düsen (12), vorzugsweise Flachstrahldüsen (12a) oder Rundstrahldüsen (12b) oder eine Kombination hiervon umfasst.

10. Rheologiemessverfahren unter Verwendung einer Rheologiemessvorrichtung gemäß einem der Ansprüche 1 bis 9, umfassend die folgenden Schritte:
a) Einbringen der Rheologiemessvorrichtung (1) in ein zu vermessendes Mischgut,
b) Bewegen des Rheologiemesswerkzeugs (2) in das zu vermessende Mischgut, wobei die Eintauchtiefe des Messkörpers (2a) in das zu vermessende Mischgut durch den Abstandssensor (6) gesteuert wird,
c) Beaufschlagen des Messkörpers (2a) mit einem Drehmoment, welches durch den Motor (3) erzeugt wird,
d) Erhöhen des Drehmoments, bis sich der Messkörper (2a) zu drehen beginnt,
e) Erfassen der Drehbewegung des Messkörpers (2a) durch den Drehzahlsensor (5),
f) weiteres Erhöhen des Drehmoments, bis ein vorbestimmtes Messdrehmoment erreicht ist, dabei Aufzeichnen der Drehzahl und des Drehmoments des Messkörpers (2a),
g) Bestimmender rheologischen Eigenschaften des zu vermessenden Mischguts.

11. Rheologiemessverfahren unter Verwendung einer Rheologiemessvorrichtung gemäß einem der Ansprüche 1 bis 9, umfassend die folgenden Schritte:
a) Einbringen der Rheologiemessvorrichtung (1) in ein zu vermessendes Mischgut,
b) Bewegen des Rheologiemesswerkzeugs (2) in das zu vermessende Mischgut, wobei die Eintauchtiefe des Messkörpers (2a) in das zu vermessende Mischgut durch den Abstandssensor (6) gesteuert wird,
c) Rotieren des Messkörpers (2a) durch den Motor (3) mit einer Drehzahl,
d) Erfassen des Drehmoments des Messkörpers (2a) durch den Drehmomentsensor (4),
e) weiteres Erhöhen der Drehzahl, bis eine vorbestimmte Messdrehzahl erreicht ist, dabei Aufzeichnen der Drehzahl und des Drehmoments des Messkörpers (2a),
f) Bestimmender rheologischen Eigenschaften des zu vermessenden Mischguts.

12. Rheologiemessverfahren gemäß Anspruch 10 oder 11, ferner die folgenden Schritte aufweisend:
h) Ausfahren der Rheologiemessvorrichtung (1) aus dem zu vermessenden Mischgut,
i) Reinigen der Rheologiemessvorrichtung (1) durch die Reinigungsvorrichtung (11).

13. Verwendung einer Rheologiemessvorrichtung (1) gemäß einem der Ansprüche 1 bis 9 für eine Rheologiemessung eines Mischguts in einem Mischer, vorzugsweise einem Betonmischer.
